# EUROPÄISCHE PATENTANMELDUNG

(11) **EP 0 620 000 A2**
(43) Veröffentlichungstag der Anmeldung: **19.10.1994**
(21) Anmeldenummer: 93119913.7
(22) Anmeldetag: 10.12.1993
(51) Int. Cl.: A61K 7/48, A61K 7/42, A61K 7/40

(54) **Kosmetische und/oder pharmazeutische Verwendung von N-Acylalkanolaminen**

(30) Priorität: 18.12.1992 DE 4242959; 18.12.1992 DE 4242960; 26.01.1993 DE 4302074; 24.02.1993 DE 4305552
(71) Anmelder: RHONE-POULENC RORER GMBH, 50792 Köln (DE)
(72) Erfinder: Ghyczy, Miklos, D-50933 Köln (DE); Nissen-Zoufal, Brigitte, D-53347 Alfter (DE); Gehring, Wolfgang, D-76467 Bietigheim (DE)
(74) Vertreter: Döring, Wolfgang, Dr. Ing.

(57) **Zusammenfassung**

Es wird die Verwendung von mindestens einem N-Acylalkanolaminderivat der allgemeinen Formel I 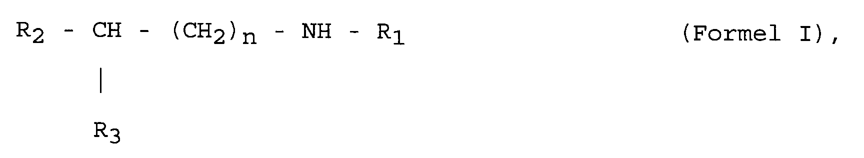
in kosmetischen und/oder pharmazeutischen Zubereitungen zur Verhinderung von durch Lichtstrahlung verursachten Hautschäden und/oder zur Behandlung von durch Licht und/oder Wärme oder Insektenstichen hervorgerufenen Hautschäden beschrieben.

## Beschreibung

Die vorliegende Erfindung betrifft die Verwendung von N-Acyl-Alkanolaminen in pharmazeutischen und/oder kosmetischen Zubereitungen.

Die Verwendung von N-Acyl-Ethanolaminen in Produkten zur Haarpflege ist bekannt. So beschreibt beispielsweise eine Veröffentlichung von Goldemberg in J.Soc.Cosmet. Chem., 30, 1979, S. 415 - 427 die Anwendung von Acetyl-Ethanolaminen (MEA), um hierdurch Augenreizungen, hervorgerufen durch tensidische Bestandteile in Shampoos, zu reduzieren.

Die orale Anwendung von N-Oleyl-Phosphatidylethanolaminen als pharmazeutisches Mittel besitzt gemäß der DE 27 56 866 A1 eine deutlich antiarteriosklerotische bzw. antilipämische Wirkung, was anhand von tierexperimentellen Prüfungen bewiesen werden konnte.

Um durch Lichtstrahlung, insbesondere durch Sonnenstrahlung, verursachte Hautschäden zu verhindern oder um durch Licht und/oder Wärme oder Insektenstichen hervorgerufene Hautschäden zu behandeln, sind eine Reihe von pharmazeutischen und/oder kosmetischen Produkten bekannt.

So weisen beispielsweise die herkömmlichen bekannten Sonnenschutzmittel, die eine Hautschädigung durch Sonneneinstrahlung verhindern sollen, als Wirkstoffe solche Substanzen auf, die die UV-Strahlung im Sonnenlicht absorbieren oder reflektieren. Zu den absorbierenden Substanzen gehören beispielsweise p-Aminobenzoesäure oder Derivate davon, Salicylate, Anthranilate, Cinnamate, Benzophenone oder Campherderivate, während die reflektierenden Sonnenschutzmittel meistens Titandioxid oder Zinkoxid als Wirkstoff enthalten.

Um eine durch Licht, Wärme oder Insektenstichen hervorgerufene Hautschädigung bzw. -reizung zu behandeln, werden äußerlich, insbesondere bei schwerwiegenden Hautschädigungen, Corticosteroide-Cremes oder oral Acetylsalicylsäure, Indometacin oder ebenfalls Corticosteroide eingesetzt.

Die zuvor genannten bekannten pharmazeutischen bzw. kosmetischen Produkte weisen den Nachteil auf, daß sie teilweise in ihrer Wirkung nur begrenzt sind und somit hochdosiert werden müssen, was dann wiederum zu unerwünschten Hautreizungen führt, oder andere, noch gravierendere Nebenwirkungen, wie beispielsweise Magengeschwüre, Hautatrophien, Steroidakne, Candidabefall der Schleimhäute oder psychische Störungen bei dauerhafter Anwendung von Corticosteroiden hervorrufen.

Der vorliegenden Erfindung liegt die Aufgabe zugrunde, einen Wirkstoff für kosmetische und/oder pharmazeutische Zubereitungen zur Verfügung zu stellen, mit dem sowohl lichtstrahlungsbedingte Hautschäden besonders wirksam verhindert werden können und mit dem gleichzeitig noch durch Licht und/oder Wärme oder Insektenstiche hervorgerufene Hautschäden schnell und wirksam therapiert werden können.

Diese Aufgabe wird erfindungsgemäß durch die Verwendung von mindestens einem N-Acyl-Alkanolamin-Derivat als Wirkstoff in kosmetischen und/oder pharmazeutischen Zubereitungen gelöst, wobei das zuvor genannte N-Acyl-Alkanolamin-Derivat einen chemischen Aufbau aufweist, wie dieser in der allgemeinen Formel I nachfolgend wiedergegeben ist.

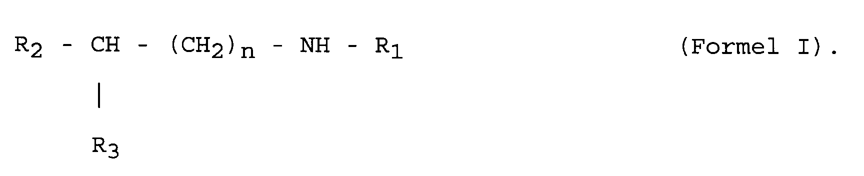

In dieser allgemeinen Formel I bedeuten
- R₁: der Acylrest einer gesättigten C₁ - C₁₈-Carbonsäure und/oder eines gesättigten C₁ - C₁₈-Carbonsäurederivates und/oder
der Acylrest einer ungesättigten C₃ - C₁₈-Carbonsäure und/oder eines ungesättigten C₃ - C₁₈-Carbonsäurederivates;
- R₂: eine Hydroxy-Gruppe und/oder eine Estergruppe;
- R₃: Wasserstoff und/oder der C₁ - C₄-Alkylrest; und
- n: eine ganze Zahl zwischen 0 und 5.

Überraschend konnte festgestellt werden, daß durch die zuvor wiedergegebene erfindungsgemäße Verwendung eines N-Acyl-Alkanolamin-Derivates als Wirkstoff in kosmetischen und/oder pharmazeutischen Zubereitungen sowohl die durch Lichtstrahlung, insbesondere durch Sonnenstrahlung, verursachten Hautschäden verhindert werden als auch bereits aufgetretene Hautschäden, die durch Licht und/oder Wärme oder Insektenstiche verursacht wurden, innerhalb von kürzester Zeit wirkungsvoll therapiert werden können.

Insbesondere konnte festgestellt werden, daß der in Formel I wiedergegebene Wirkstoff vorzugsweise bei einer topischen Anwendung Lichtdermatosen und lichtbeeinflußbare Dermatosen, wie beispielsweise Dermatitis solaris, aktinische Atrophie, Cutis rhomboidalis nuchae, Erythrosis interfollicularis colli, Morbus Favre Racouchot, Keratosen, Cornu cutaneum, Cheilitis actinica, Sommerprurigo, Mallorca-Akne, phototoxische Dermatitis, Wiesengräserdermatitis, photoallergische Dermatitis, polymorphe Lichtdermatosen, Hydroa racciniformia, Xeroderma pigmentosum, Lupus erythematodes, rosaceaartige Dermatitis, seborrhoische Ekzeme und Lichen ruber planus, sowie mit den zuvor genannten Hautschädigungen bzw. Hauterkrankungen verbundene Begleiterscheinungen, wie insbesondere das Austrocknen, das Rauhwerden, die Rötung und die Faltenbildung sowie die vorzeitige Alterung der Haut, verhindert werden können.

Desweiteren konnte überraschend festgestellt werden, daß man mit einer therapeutischen Zubereitung, die mindestens ein N-Acyl-Alkanolamin-Derivat der in der Formel I angegebenen chemischen Zusammensetzung als Wirkstoff enthält, die vorstehend genannten Hautschäden bzw. -erkrankungen und -reizungen besonders wirkungsvoll behandeln kann, so daß eine rasche Heilung auftritt.

Auch bei Insektenstichen, so zum Beispiel Bienenstichen, Wespenstichen, Hornissenstichen, Mückenstichen, Milbenstichen, Zeckenbissen, Sandflohbissen, Stechfliegenbissen und Bremsenbissen, konnte festgestellt werden, daß bereits nach kürzester Zeit nach Auftragen einer pharmazeutischen Zubereitung, die den in Formel I wiedergegebenen Wirkstoff enthält, ein deutlicher Rückgang der durch die zuvor genannten Stiche bzw. Bissen hervorgerufenen Rötungen, Schwellungen bzw. Quaddelbildungen auftritt, während gleichzeitig der durch den Insektenstich hervorgerufene Schmerzreiz bzw. Juckreiz deutlich reduziert wird.

Darüber hinaus weist die erfindungsgemäße Verwendung des in Formel I wiedergegebenen Wirkstoffes in einer entsprechenden kosmetischen und/oder pharmazeutischen Zubereitung den weiteren Vorteil auf, daß selbst bei empfindlichen Patienten keine unerwünschte Reizung, Rötung der Haut oder sonstige Nebenwirkungen bei einer topischen Anwendung festgestellt werden konnte. Ferner sind die erfindungsgemäß verwendeten Wirkstoffe nicht toxisch, so daß sie völlig unbedenklich sind und auch von empfindlichen Patienten besonders gut vertragen werden.

Darüber hinaus stellt es kein Problem dar, die in der Formel I allgemein wiedergegebenen Wirkstoffe in Wasser zu lösen, zu dispergieren oder zu emulgieren, so daß die Herstellung einer lagerstabilen kosmetischen bzw. pharmazeutischen Zubereitung keine Probleme bereitet. Auch ist die Verfügbarkeit der in Formel I allgemein beschriebenen Wirkstoffe gegeben, wobei vorzugsweise insbesondere die nachfolgend noch beschriebenen Phosphorsäureester aus Naturprodukten isoliert werden können.

Wie bereits vorstehend erwähnt ist, wird die pharmazeutische bzw. kosmetische Zubereitung, die den in Formel I allgemein wiedergegebenen Wirkstoff enthält, vorzugsweise in einer solchen Darreichungsform aufgemacht, die zur topischen Anwendung geeignet ist. Dies bedeutet, daß für die topische Anwendung eine Lösung, Suspension, Emulsion oder Dispersion der zuvor genannten Wirkstoffe bzw. Wirkstoffkombinationen gemäß der allgemeinen Formel I in Form von Tropfen, Tinkturen oder Sprays als pharmazeutische bzw. kosmetische Zubereitung angeboten wird. Als halbfeste Zubereitungen kommen beispielsweise Gele, Salben, Cremes oder Schäume in Frage, während unter die feste Zubereitungen beispielsweise Pulver, Puder, Granulate, Pellets und Mikrokapseln fallen.

Wird die beanspruchte pharmazeutische bzw. kosmetische Zubereitung in der Form einer flüssigen Darreichungsform verwendet, so empfiehlt es sich, hierfür möglichst solche Verdünnungsmittel zu verwenden, die die Haut nicht reizen. Dies trifft beispielsweise auf Wasser, einwertige Alkohole, insbesondere Ethanol, Isopropanol oder n-Propanol, mehrwertige Alkohole, insbesondere Glycerin und/oder Propandiol, Polyglykole, insbesondere Polyethylenglykole und/oder Miglyol, Glycerinformal, Dimethylisosorbit, natürliche und synthetische Öle und/oder Ester, zu.

Für die Herstellung von halbfesten Darreichungsformen, wie beispielsweise Gele, Salben, Cremes und Schäume, eignen sich neben den zuvor genannten Verdünnungsmitteln zusätzlich noch Grundmassen, wie beispielsweise Bentonit, Veegum, Guarmehl und/oder Cellulosederivate, insbesondere Methylcellulose und/oder Carboxymethylcellulose. Ebenso kommen als Grundmasse anstelle der zuvor genannten Grundmassen oder zusätzlich zu den zuvor genannten Grundmassen Polymere aus Vinylalkohole, Vinylpyrolidone, Alginate, Pektine, Polyacrylate, feste und/oder flüssige Polyethylenglykole, Paraffine, Fettalkohole, Vaselin, Wachse, Fettsäuren und/oder Fettsäureester in Frage.

Für die Herstellung von festen Darreichungsformen, wie beispielsweise die zuvor genannten Pulver, Puder, Granulate, Pellets oder Mikrokapseln, besteht die Möglichkeit, hier als Bindemittel beispielsweise kolloidale Kieselsäure, Talkum, Milchzucker, Stärkepulver, Zucker, Cellulosederivate, Gelatine, Metalloxide und/oder Metallsalze zu verwenden, wobei bevorzugt Zinkoxid und/oder Titandioxid eingesetzt wird, da diese zuletzt genannten speziellen Metalloxide zusätzlich noch die Eigenschaft haben, die Schutzwirkung einer entspechend konfektionierten pharmazeutischen oder kosmetischen Zubereitung in bezug auf Sonneneinstrahlung zu erhöhen.

Darüber hinaus können die zuvor beschriebenen Darreichungsformen wahlweise noch weitere Zusätze, wie beispielsweise Konservierungsmittel, Stabilisatoren, Tenside, Emulgatoren, Penetrationsförderer, Spreitungsmittel und/oder Treibmittel, enthalten.

Eine besonders geeignete Ausführungsform der erfindungsgemäßen Zubereitung, die insbesondere zur topischen Anwendung geeignet ist, weist als weiteren Zusatz Phospholipide, insbesondere Soja-Phospholipide, auf. Hierfür werden vorzugsweise solche Soja-Phospholipidfraktionen verwendet, die mindestens 80 Gew.% Phosphatidylcholin enthalten, wobei die Konzentration des phospholipidischen Zusatzes in der anwendungsfertigen kosmetischen bzw. pharmazeutischen Zubereitung je nach Darreichungsform und Anwendungsgebiete zwischen 1 Gew.% und 25 Gew.%, vorzugsweise zwischen 2 Gew.% und 15 Gew.%, variiert.

Wird die beanspruchte pharmazeutische bzw. kosmetische Zubereitung als Sonnenschutzmittel verwendet, empfiehlt es sich inbesondere in den Fällen, in denen die Zubereitung einen besonders hohen Sonnenschutzfaktor aufweisen soll, zusätzlich noch zu dem mindestens einen Wirkstoff eine Substanz zu verwenden, die eine lichtabsorbierende Wirkung besitzt. Hierfür kommen solche Substanzen in Frage, wie diese eingangs beim Stand der Technik aufgeführt sind, so beispielsweise Derivate der p-Aminobenzoesäure, Octyldimethyl-p-Aminobenzoesäure, Anthranilate, Cinnamate, Benzophenone und/oder Campherderivate, wobei jedoch im Unterschied zu den bekannten Sonnenschutzmittel die Konzentration dieser lichtabsorbierenden Substanzen in der pharmazeutischen bzw. kosmetischen Zubereitung erheblich reduziert wird, vorzugsweise auf eine Konzentration, die zwischen 50 und 70 % unterhalb der Konzentration liegt, die in bekannten Sonnenschutzmitteln enthalten ist. Dies hängt damit zusammen, daß der mindestens eine Wirkstoff der allgemeinen Formel I offensichtlich mit den bekannten lichtabsorbierenden bzw. lichtreflektierenden Substanzen eine synergistische Wirkung besitzt, so daß diese bekannten lichtabsorbierenden bzw. lichtreflektierenden Substanzen unter Beihaltung eines identischen und sogar verbesserten Lichtschutzfaktors erheblich reduziert werden können.

Eine weitere Variante der erfindungsgemäßen Verwendung sieht vor, daß mindestens ein Wirkstoff eingesetzt wird, bei der in der Formel I R₂ eine Hydroxy-Gruppe bedeutet. Mit anderen Worten weist eine derartige pharmazeutische bzw. kosmetische Zubereitung als mindestens einen Wirkstoff ein N-Acyl-Alkanolamin auf.

Insbesondere dann, wenn bei der erfindungsgemäßen Verwendung mindestens ein Wirkstoff verwendet wird, bei dem in der vorstehend genannten allgemeinen Formel I R₃ Wasserstoff und n = 1 bedeuten, lassen sich besonders gute Wirksamkeiten in bezug auf die Verhinderung bzw. Behandlung der vorstehend genannten Hautschädigungen erreichen. Eine derartige Zubereitung weist dann mindestens einen Wirkstoff auf, der ein N-Acyl-Ethanolamin umfaßt. Ebenso bevorzugt wird ein Wirkstoff eingesetzt, der N-Oleoyl-Ethanolamin, N-Linolenoyl-Ethanolamin, N-Acyl-Ethanolamin und/oder N-Acyl-2-Hydroxy-Propylamid verwendet, wobei die zuletzt genannten beiden Produkte als Acylreste Fettsäuren aus Kokosfett (Kokosöl) und/oder Palmöl enthalten.

Wie bereits eingangs erwähnt ist, wird bei der erfindungsgemäßen Verwendung eine Zubereitung eingesetzt, die als mindestens einen Wirkstoff ein N-Acyl-Alkanolamin-Derivat (Formel I) enthält und bei dem R₁ in der Formel I den Acylrest einer gesättigten C₁ - C₁₈-Carbonsäure und/oder eines gesättigten C₁ - C₁₈-Carbonsäurederivates und/oder den Acylrest einer ungesättigten C₃ - C₁₈-Carbonsäure und/oder eines ungesättigten C₃ - C₁₈-Carbonsäurederivates darstellt.

Durch die gewählte Formulierung und/oder wird zum Ausdruck gebracht, daß das N-Acyl-Alkanolamin-Derivat auch als Mischung vorliegen kann, wobei dann z.B. ein erster Mischungsbestandteil einen chemischen Aufbau besitzt, bei dem R₁ und/oder R₆ (Formel I - IV) der Acylrest einer gesättigten C₁-C₁₈-Carbonsäure ist, ein zweiter Mischungsbestandteil einen chemischen Aufbau aufweist, bei dem R₁ und/oder R₆ der Acylrest einer ungesättigten C₃-C₁₈-Carbonsäure ist, ein dritter Mischungsbestandteil einen Aufbau aufweist, bei dem R₁ und/oder R₆ der Acylrest eines gesättigten C₁-C₁₈-Carbonsäurederivates ist, und/oder ein vierter Mischungsbestandteil einen Aufbau hat, bei dem R₁ und/oder R₆ der Acylrest eines ungesättigten C₃-C₁₈-Carbonsäurederivates ist, wobei eine zusätzliche Variation der Mischung durch eine entsprechende Veränderung von n, R₂, R₃, R₄ und R₅ im Rahmen der vorstehend angegebenen Bedeutungen möglich ist.

Insbesondere bietet es sich an, die nachfolgend aufgeführten Derivate oder Mischungen hiervon zu verwenden:

Essigsäure-2-Hydroxy-Ethylamid, Laurinsäure-2-Hydroxy-Ethylamid, Stearinsäure-2-Hydroxy-Ethylamid, Linolsäure-2-Hydroxy-Ethylamid, Palmitinsäure-2-Hydroxy-Ethylamid, Laurinsäure-2-Hydroxy-Propylamid, Linolsäure-2-Hydroxy-Propylamid, Ölsäure-2-Hydroxy-Propylamid und die entsprechenden 2-Hydroxy-Ethylamid- und/oder 2-Hydroxy-Propylamid-Derivate, die als Acylreste Fettsäuren aus Kokosfett (Kokosöl) und/oder Palmöl enthalten.

Vorzugsweise werden jedoch solche Wirkstoffe bei der erfindungsgemäßen Verwendung eingesetzt, bei denen in Formel I R₁ für den Acylrest der Palmitinsäure, der Stearinsäure, der Ölsäure, der Essigsäure, der Propionsäure und/oder der 2-Hydroxy-Propionsäure steht.

Weiterhin werden besonders bevorzugt solche Wirkstoffe für die Herstellung der beanspruchten Zubereitung eingesetzt, bei denen in der Formel I R₂ für eine Hydroxy-Gruppe und R₃ für eine Methylgruppe stehen, wobei in diesem Fall n = 1 ist.

Eine besonders geeignete Verwendung erlaubt eine pharmazeutische bzw. kosmetische Zubereitung, die mindestens einen Wirkstoff aufweist, bei dem in der Formel I R₂ für ein Phosphorsäureester steht. Hierbei hat sich aufgrund der Untersuchungen zur Schutzwirkung und Therapie ein Phosphorsäureester mit hoher Wirksamkeit herauskristallisiert, der einen chemischen Aufbau aufweist, wie dieser durch die nachfolgend wiedergegebenen allgemeine Formel II charakterisiert ist. 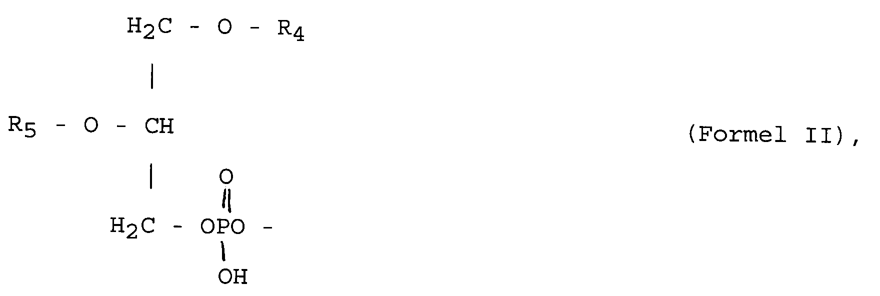
In der Formel II sind R₄ und R₅ gleich oder verschieden und bedeuten Wasserstoff und/oder der Acylrest einer gesättigten und/oder ungesättigten C₁₆-C₁₈-Carbonsäure und/oder der Acylrest eines gesättigten und/oder ungesättigten C₁₆-C₁₈-Carbonsäurederivates, so daß unter Verwendung dieses Phosphorsäureesters die beanspruchte Zubereitung eine chemische Formel aufweist, wie diese durch die Formel II a nachfolgend angegeben ist. 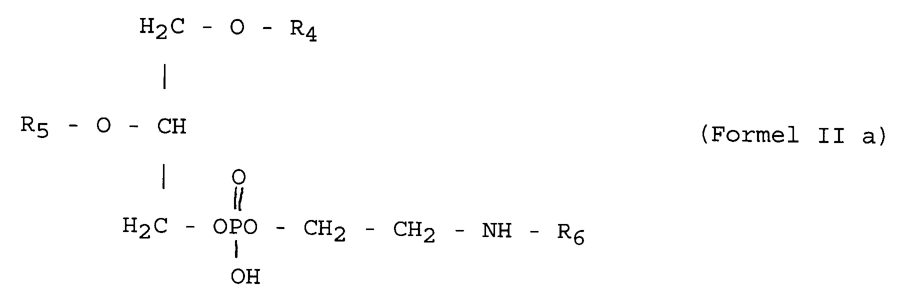
In dieser Formel bedeuten R₆ der Acylrest einer gesättigten C₁-C₁₈-Carbonsäure und/oder eines gesättigten C₁-C₁₈-Carbonsäurederivates und/oder der Acylrest einer ungesättigten C₃-C₁₈-Carbonsäure und/oder eines ungesättigten C₃-C₁₈-Carbonsäurederivates; während R₄ und R₅ gleich oder verschieden sind und für Wasserstoff und/oder den Acylrest einer gesättigten und/oder ungesättigten C₁₆-C₁₈-Carbonsäure und/oder den Acylrest eines gesättigten und/oder ungesättigten C₁₆-C₁₈-Carbonsäurederivates stehen.

Wie bereits vorstehend wiederholt erwähnt ist, kann für die beanspruchte Verwendung eine solche kosmetische bzw. pharmazeutische Zubereitung eingesetzt werden, die einen Wirkstoff oder eine Mischung von Wirkstoffen enthält. Besonders geeignet ist es, wenn die Zubereitung eine Mischung von Wirkstoffen enthält, wobei diese Mischung einen ersten Wirkstoff auf der Basis eines N-Acyl-Ethanolamins der allgemeinen Formel III

HO - CH₂ - CH₂ - NH - R₁ (Formel III)

und einen zweiten Wirkstoff auf der Basis eines N-Acyl-Phosphatidylethanolamins der allgemeinen Formel IV 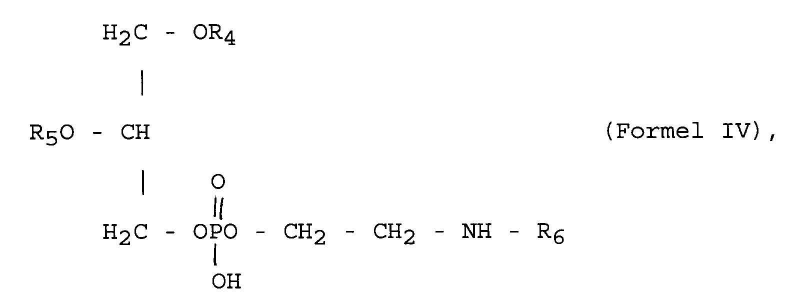
enthält, wobei in Formel IV R₄ und R₅ gleich oder verschieden sind und Wasserstoff und/oder der Acylrest einer gesättigten und/oder ungesättigten C₁₆-C₁₈-Carbonsäure und/oder der Acylrest eines gesättigten und/oder ungesättigten C₁₆-C₁₈-Carbonsäurederivates, vorzugsweise für die vorstehend genannten konkreten Acylreste stehen, und R₁ (Formel III) und R₆ (Formel IV) der Acylrest einer gesättigten C₁ - C₁₈-Carbonsäure und/oder eines gesättigten C₁ - C₁₈-Carbonsäurederivates und/oder der Acylrest einer ungesättigten C₃ - C₁₈-Carbonsäure und/oder eines ungesättigten C₃ - C₁₈-Carbonsäurederivaten bedeuten.

Besonders gute Ergebnisse in bezug auf die Verhinderung und Behandlung von Hautschäden lassen sich dann erreichen, wenn als zweiter Wirkstoff N-Acetyl-Phosphatidylethanolamin, N-Palmityl-Phosphatidylethanolamin und/oder N-Oleyl-Phosphatidylethanolamin jeweils allein oder in Mischung verwendet wird. Vorzugsweise werden die zuvor konkret genannten Ethanolamine aus natürlichem Material, insbesondere aus Sojabohnen, isoliert.

Abhängig von der jeweiligen Verwendung richtet sich die Konzentration des Wirkstoffes bzw. der Wirkstoffmischung in der kosmetischen bzw. pharmazeutischen Zubereitung. Üblicherweise variiert diese Konzentration zwischen 0,5 Gew.% und 25 Gew.%, vorzugsweise zwischen 1 Gew.% und 10 Gew.%, bezogen auf die Masse der anwendungsfertigen Zubereitung.

Wird bei der erfindungsgemäßen Verwendung mit einer Zubereitung gearbeitet, die den vorstehend angegeben ersten Wirkstoff gemäß der Formel III und den zweiten Wirkstoff gemäß der Formel IV aufweist, so variiert vorzugsweise das Massenverhältnis von erstem Wirkstoff (Formel III) zu zweitem Wirkstoff (Formel IV) zwischen 55:45 bis 80:20, wobei sich diese Angaben auf die anwendungsfertige Zubereitung beziehen.

Abhängig von der jeweiligen Anwendung kann die Zubereitung hautpflegende Zusätze oder solche Bestandteile enthalten, die die therapeutische Wirksamkeit erhöhen. Hierfür kommen insbesondere Lokalanästhetika, Antibiotika, Antimykotika, Antihistaminika, Steroide, UV-Filter, Vitamine, Peptide, Kollagene, Hyaluronsäure und/oder Pflanzenextrakte in Frage.

Vorteilhafte Weiterbildungen der erfindungsgemäßen Verwendung gehen aus den Unteransprüchen hervor.

Die erfindungsgemäße Verwendung wird nachfolgend anhand von Ausführungsbeispielen näher erläutert.

Es wurden 12 Zubereitungen zur topischen Anwendung hergestellt, die die nachfolgend wiedergegebenen Zusammensetzungen aufwiesen:

### Zubereitung 1

- Phase A: 5,00 g N-Acetylethanolamin
25,00 g Propylenglycol
10,00 g Ethanol
18,00 g Wasser
- Phase B: 35,00 g Carbopol 940 - 2 % in Wasser
- Phase C: 7,00 g Triethanolamin - 10 %ig in Wasser

Die Phasen wurden hergestellt und in der Reihenfolge A + B + C und durch einen schnell laufenden Rührer homogenisiert.

### Zubereitung 2

- Phase A: 8,00 g Crodawax GP 200 (Mischung von PEG-Estern, Cetylstearylalkohol)
10,00 g Paraffinöl
- Phase B: 6,00 g N-Acetylethanolamin
70,50 g Wasser
5,00 g Natrium PCA
- Phase C: 0,50 g Euxyl K 400

Die Substanzen der Phase A wurden unter Erwärmung vermischt und mit den Phasen B + C in der Reihenfolge A + B + C durch einen schnell laufenden Rührer homogenisiert.

### Zubereitung 3

1,00 g N-Acetylethanolamin
99,00 g Ethanol DAB
Die beiden Produkte wurden miteinander vermischt.

### Zubereitung 4

2,00 g N-Oleylethanolamin
10,00 g Harnstoff
20,00 g Wasser
ad 100,00 g Umquentum Cordes (Ichtiol)
Die Produkte wurden in einem schnell laufenden Rührer homogenisiert.

### Zubereitung 5

- Phase A: 10,00 g Sojaphospholipidfraktion, enthaltend
80 Gew.% Phosphatidylcholin
10 Gew.% Phosphatidsäure
5 Gew.% Phosphatidylethanolamin
3,00 g Propylenglycol
5,00 g Weizenkeimöl
1,25 g N-Oleylethanolamin
- Phase B: 0,90 g NaCl
79,15 g Wasser
- Phase C: 0,50 g Euxyl K 400
0,20 g NaOH 10 %ig

Die Phase A wurde bei 60 - 70 °C geschmolzen und mit der Phase B und dann mit der Phase C in einem schnell laufenden Rührer homogenisiert.

### Zubereitung 6

3,00 g Polyoxyethylenfettalkoholether (Brig 96™)
0,50 g N-Palmitoylethanolamin
0,40 g Cholesterin
0,07 g NaCl
ad 100,00 g Wasser
Die Mischung wurde bei 80 °C 5 Minuten lang mit Ultraschall homogenisiert.

### Zubereitung 7

10,00 g Phosphatidylcholin aus der Sojabohne
2,00 g N-Acetylethanolamin
0,70 g NaCl
ad 100,00 g Wasser
Die Mischung wurde bei 40 °C in einem schnell laufenden Rührer (Stephan UMC 5) 20 Minuten homogenisiert.

### Zubereitung 8

- Phase A: 55,00 g Wasser
14,00 g Propylenglycol
18,00 g Phosphatidylcholin (Soja)
- Phase B: 1,00 g N-Acetylphosphatidylethanolamin
2,00 g N-Acetylethanolamin
10,00 g Jojobaöl

Phase A wurde bei 40 °C in einem schnell laufenden Rührer 40 Minuten homogenisiert bis ein klares Gel entstand. Anschließend wurde die Phase B zugefügt und so lange gerührt, bis eine homogene Gesamtphase vorlag.

### Zubereitung 9

- Phase A: 5,00 g Weizenkeimöl
1,00 g N-Palmitylethanolamin
1,00 g Glycerinmonostearat (Imwitor 960)
- Phase B: 0,50 g Euxyl K 40
92,50 g Wasser

Die Produkte der Phase A wurden unter Erwärmen auf 70 °C so lange gemischt, bis eine homogene Phase vorlag. Die Phase B wurde auf 70 °C erwärmt und danach zur Phase A gegeben. Anschließend wurde unter Rühren auf Raumtemperatur abgekühlt.

### Zubereitung 10

3,00 g N-Acetylphosphatidylethanolamin
97,00 g Ethanol

### Zubereitung 11

10,00 g Sojaphospholipidfraktion (Zusammensetzung wie Zubereitung 5)
4,00 g N-Oleylphosphatidylethanolamin
0,70 g NaCl
ad 100,00 g Wasser
Das N-Oleoylphosphatidylethanolamin wurde in Wasser bei 70°C gerührt, bis eine homogene Phase vorlag. Nachdem die Sojaphospholipidfraktion hinzugefügt war, wurde 5 min. gerührt, dann wurde das NaCl hinzugegeben und weitere 50 min. gerührt. Nach Hitzesterilisierung konnte das Produkt in Ampullen abgefüllt werden.

### Zubereitung 12

- Phase A: 58,00 g Wasser
14,00 g Propylenglycol
18,00 g Phosphatidylcholin (Soja)
- Phase B: 10,00 g Jojobaöl

Die Herstellung erfolgte wie bei der Zubereitung 8.

Zum Nachweis der pharmazeutischen bzw. kosmetischen Wirksamkeit der vorstehend genannten Zubereitungen wurde der Lichtschutzfaktor (Test 1), die heilende Wirksamkeit (Test 2), die Schutzwirkung für β-Karotin (Test 3), die Schutzwirkung an lebenden Zellen (Test 4) und Fallstudien an Probanden (Test 5) durchgeführt. Die Ergebnisse dieser Untersuchungen sind nachfolgend aufgeführt:

### Test 1

Die Zubereitungen 3, 4, 8, 10, 11 und 12 wurden nach DIN 67 501 an 20 Probanden untersucht. Als Kontrolle für diese Bestimmungen des Lichtschutzfaktors (LSF) wurde ein Produkt ausgewählt, das einen deklarierten Lichtschutzfaktor von 4 besaß.

Die Ergebnisse der Messung des Lichtschutzfaktors sind der nachfolgenden Tabelle 1 zu entnehmen.

**Tabelle 1**

| Zubereitung | mittlerer gefundener LSF |
|---|---|
| Handelsprodukt | 4,5 (deklariert LSF 4) |
| 3 | 7,7 |
| 8 | 8,7 |
| 4 | 8,1 |
| 10 | 7,1 |
| 11 | 9,1 |
| 12 | 2,1 |

### Test 2

Um die heilende Wirkung der Zubereitung 8 in bezug auf Entzündungen zu beweisen, wurde eine Untersuchung an 10 gesunden Probanden durchgeführt.

Hierbei wurde eine Entzündung mittels subkutaner Applikation von Histamin ausgelöst, die dabei auftretende Rötung mittels Chromameter und die dabei auftretende Hautdurchblutung mittels Laser-Doppler-Methode quantifiziert. Diese Methode ist insoweit für die Überprüfung der heilenden Wirkung von Lichtschädigungen relevant, da die primäre Schädigung der Haut durch Licht die Freisetzung des Histamins aus den Mastzellen der Haut bewirkt. Das dabei freigesetzte Histamin seinerseits führt wiederum zu Hautentzündungen, wie diese beispielsweise auch beim Sonnenbrand auftreten.

Die zuvor beschriebene Testmethode stellt eine anerkannte Testmethode dar und ist in "Methods in Skin Research, 1985, S. 511 ff" beschrieben.

Der Verlauf der Heilung des Entzündungsvorganges wird durch die nachfolgenden beiden Abbildungen 1 und 2 wiedergegebenen.

Wie die beiden Abbildungen 1 und 2 eindeutig beweisen, zeigt die Zubereitung 8 bereits nach 15 bzw. 30 Minuten im Vergleich zur Zubereitung 12 eine signifikante Abnahme der Rötung und der Hyperämie.

### Test 3

In einem weiteren Test wurde quantifiziert, inwieweit β-Karotin zerstört wird, das auf die Haut aufgetragen wurde. β-Karotin stellt eine der wichtigsten Verbindungen dar, um lebendes Gewebe gegen eine Zerstörung, die durch Licht ausgelöst wird, zu schützen. Hierbei wurde auf den Unterarm von 10 Probanden zunächst 40 µl einer β-Karotin-Lösung (5 mg in 2,5 ml Hexan) auf eine definierte markierte große Hautstelle aufgetragen. Nach einer Verweilzeit von 10 Minuten wurden die Zubereitungen 12, 8, 10, 1, 4, 2 auf die definierten Hautstellen aufgebracht, wobei die Konzentration an der Zubereitung 3 mg/cm² betrug.

Die so behandelten definiert großen Hautflächen wurden nach einer Stunde mit einer Ultra-Vita-UVL-Lampe 3 Minuten lang in einem Abstand von 50 cm bestrahlt. Unmittelbar danach wurde die Hautfläche mit 1 ml Isopropanol 1 Minute lang extrahiert. In dem Extrakt wurde das β-Karotin hochdruckflüssigkeitschromatographisch quantitativ bestimmt. Hierfür wurde Methanol/n-Hexan (V:V 75:25) als Eluent bei einer Flow-Rate von 1 ml/min unter Anwendung einer RP 18,3 µ 4,6 x 75 mm (Ultrasphere Beckman-Säule) eingesetzt. Am Auslaß der Säule war ein UV-Detektor vorgesehen, der auf eine Wellenlänge von 436 nm eingestellt war.

Die Zerstörung des β-Karotins infolge der Lichteinstrahlung geht aus der nachfolgenden Tabelle hervor.

| Zubereitung | relatives prozentuales Maß der Zerstörung |
|---|---|
| 12 | 100 |
| 8 | 24 |
| 10 | 31 |
| 1 | 20 |
| 4 | 34 |
| 2 | 22 |

### Test 4

Dieser Test zeigt die Schutzwirkung der Zubereitung 7 sowie der Zubereitung 7 a gegenüber Licht an lebenden Hautzellen.

Die Zubereitung 7 a wurde wie die Zubereitung 7 hergestellt, jedoch wurden abweichend hiervon die in der Zubereitung 7 enthaltenen 2 g N-Acetylethanolamin durch Wasser ersetzt, so daß die Zubereitung 7 a
10,00 g Phosphatidylcholin aus der Sojabohne
0,70 g NaCl
ad 100,00 g Wasser
enthielt.

Bei diesem Test wurden Hautkeratinozyten und Humanhautfibroblasten eine UVA-Behandlung ausgesetzt und die Zahl der lebenden Zellen in Abhängigkeit der Zeit ausgewertet.

Die Versuchsbedingungen waren wie folgt:

### 4.1. Wirkung von UVA-Strahlung auf Human-Hautfibroblasten

- Material:: Fibroblastenkulturen von menschlicher Haut in modifiziertem Eagle Medium mit 10 % fötalem Kälberserum 32 °C
- Versuchsaufbau:: Überführung in 5 Petrischalen, Zahl der Zellen (2 x 10⁴), UVA-Bestrahlung: Ultravitalux-Lampen (30 min, 24 h)

Die folgende Tabelle gibt die Werte für die relative Zahl der lebenden Zellen wieder.

| | Zubereitung 7 a | Zubereitung 7 | |
|---|---|---|---|
| | | 0,5 mg/ml | 1,0 mg/ml |
| 30 min | 75 | 80 | 80 |
| 24 h | 45 | 75 | 75 |

### 4.2. Wirkung von UVA-Strahlung auf Humankeratinozyten

- Material:: Menschliche Keratinozyten der Oberhaut. Kultivierung in niedrigem Calcium-Medium (keratinocyte growth medium, San Diego, Californien (USA); 5 % CO₂/100 % Luftfeuchtigkeit) Überführung in 5 Petrischalen, Zahl der Zellen (4-5 x 10⁵) UVA-Bestrahlung: Ultravitalux-Lampen (30 min, 24 h)
- Messungen:: Zahl der lebenden Zellen durch Trypan-Blau-Ausschlußtest

Die folgende Tabelle gibt die Werte für die relative Zahl der lebenden Zellen wieder.

| | Zubereitung 7 a | Zubereitung 7 | |
|---|---|---|---|
| | | 0,5 mg/ml | 1,0 mg/ml |
| 30 min | 70 | 85 | 90 |
| 24 h | 40 | 70 | 70 |

### Test 5 - Fallstudie

### 5.1. Fallstudie Verbrennung

In einer Fallstudie an 10 Probanden, die Verbrennungen 1. und 2. Grades zeigten, wurde die Wirksamkeit der Zubereitung 8 im Vergleich zur Zubereitung 12 bewiesen. Hierbei wurden ausgewählte Verbrennungsbereiche mit einer Hautgröße von 4 cm² markiert und mit der Zubereitung 8 behandelt. Der zeitliche Verlauf der Heilung zeigte eindeutig, daß bei den Hautbereichen, die mit der Zubereitung 8 behandelt waren, die Zeit bis zur erfolgten Heilung 30 % bis 60 % kürzer waren als bei den Bereichen, die mit der Zubereitung 12 behandelt waren.

### 5.2. Fallstudie - Insektenstiche

In einer weiteren Fallstudie wurde die Wirksamkeit der Zubereitung 8 am Beispiel von Insektenstichen untersucht. Hierbei wurde bei 4 Probanden, die auf den Armen bzw. den Beinen zeitgleich offensichtlich drei bis sechs Mückenstiche erhalten hatten, ein Teil dieser Mückenstiche mit der Zubereitung 8 und ein anderer Teil der Mückenstiche mit der Zubereitung 12 behandelt. Bei den mit der Zubereitung 8 behandelten Mückenstichen zeigte sich bereits nach einer kurzen Zeit (15 - 30 Minuten) ein deutlicher Rückgang der Rötung und Schwellung sowie ein Nachlassen des Juckreizes, während die mit der Zubereitung 12 behandelten Mückenstiche auch noch nach 2 bis 4 Stunden deutlich gerötet und erhaben waren und den unangenehmen Juckreiz zeigten.

## Patentansprüche

1. Verwendung von mindestens einem N-Acyl-Alkanolamin-Derivat der allgemeinen Formel I als Wirkstoff in kosmetischen und/oder pharmazeutischen Zubereitungen zur Verhinderung von durch Lichtstrahlung verursachten Hautschäden und/oder zur Behandlung von durch Licht und/oder Wärme oder Insektenstichen hervorgerufenen Hautschäden, wobei in Formel I 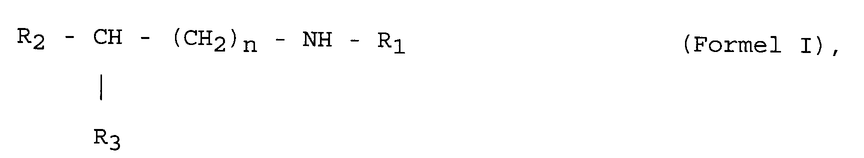
R₁ der Acylrest einer gesättigten C₁ - C₁₈-Carbonsäure und/oder eines gesättigten C₁ - C₁₈-Carbonsäurederivates und/oder
der Acylrest einer ungesättigten C₃ - C₁₈-Carbonsäure und/oder eines ungesättigten C₃ - C₁₈-Carbonsäurederivates;
R₂ eine Hydroxy-Gruppe und/oder eine Estergruppe;
R₃ Wasserstoff und/oder ein C₁ - C₄-Alkylrest; und
n eine ganze Zahl zwischen 0 und 5 bedeuten.

2. Verwendung nach Anspruch 1, wobei die Zubereitung eine zur topischen Anwendung geeignete Darreichungsform aufweist.

3. Verwendung nach Anspruch 1 oder 2, wobei mindestens ein Wirkstoff eingesetzt wird, bei dem in der Formel I
R₂ eine Hydroxy-Gruppe
bedeutet.

4. Verwendung nach einem der vorangehenden Ansprüche, wobei mindestens ein Wirkstoff verwendet wird, bei dem in der Formel I
R₃ Wasserstoff und
n gleich 1 ist.

5. Verwendung nach einem der vorangehenden Ansprüche, wobei mindestens ein Wirkstoff eingesetzt wird, bei dem in der Formel I
R₁ der Acylrest der Essigsäure, Propionsäure, 2-Hydroxypropionsäure, Palmitinsäure, der Stearinsäure und/oder der Ölsäure
ist.

6. Verwendung nach einem der vorangehenden Ansprüche, wobei mindestens ein Wirkstoff eingesetzt wird, bei dem in der Formel I
R₂ eine Hydroxy-Gruppe;
R₃ eine Methyl-Gruppe; und
n gleich 1
bedeuten.

7. Verwendung nach einem der vorangehenden Ansprüche, wobei mindestens ein Wirkstoff eingesetzt wird, bei dem in der Formel I
R₂ ein Phosphorsäureester
ist.

8. Verwendung nach Anspruch 7, wobei der Phosphorsäureester (R₂) die allgemeine Formel II 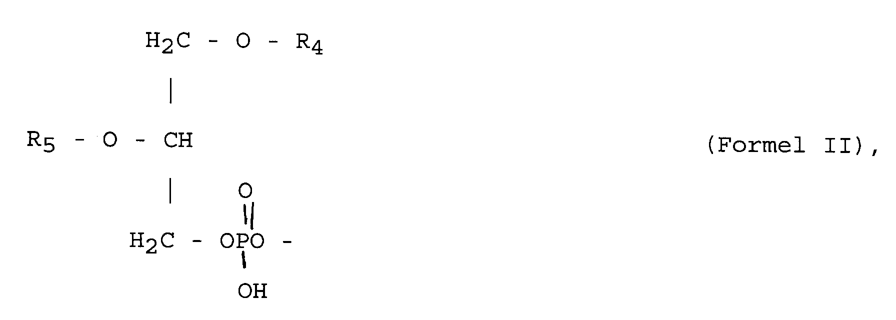 aufweist und R₄ und R₅ gleich oder verschieden sind und Wasserstoff und/oder der Acylrest einer gesättigten und/oder ungesättigten C₁₆-C₁₈-Carbonsäure und/oder der Acylrest eines gesättigten und/oder ungesättigten C₁₆-C₁₈-Carbonsäurederivates bedeuten.

9. Verwendung nach einem der vorangehenden Ansprüche, wobei eine Mischung von Wirkstoffen, die als ersten Wirkstoff ein Ethanolamin der allgemeinenen Formel III
HO - CH₂ - CH₂ - NH - R₁ (Formel III)
und als zweiten Wirkstoff ein Ethanolamin der allgemeinen Formel IV enthält 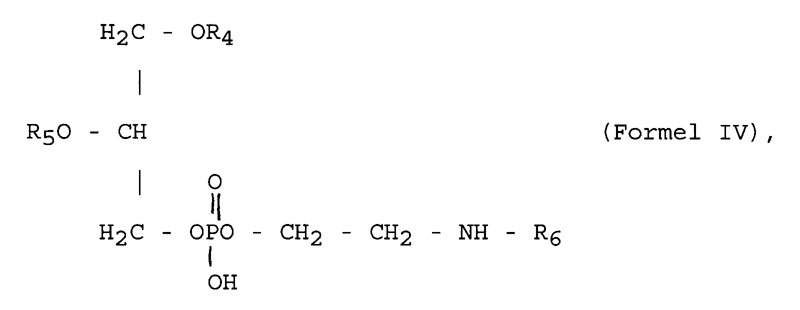 eingesetzt wird und wobei R₄ und R₅ gleich oder verschieden sind und Wasserstoff und/oder der Acylrest einer gesättigten und/oder ungesättigten C₁₆-C₁₈-Carbonsäure und/oder der Acylrest eines gesättigten und/oder ungesättigten C₁₆-C₁₈-Carbonsäurederivates; und
R₁ und R₆ gleich oder verschieden sind und der Acylrest einer gesättigten C₁ - C₁₈-Carbonsäure und/oder eines gesättigten C₁ - C₁₈-Carbonsäurederivates
und/oder
der Acylrest einer ungesättigten C₃ - C₁₈-Carbonsäure und/oder eines ungesättigten C₃ - C₁₈-Carbonsäurederivates
bedeuten.

10. Verwendung nach einem der vorangehenden Ansprüche, wobei der mindestens eine Wirkstoff bzw. die Wirkstoffmischung in einer Konzentration zwischen 0,5 Gew.% und 25 Gew.%, vorzugsweise in einer Konzentration zwischen 1 Gew.% und 10 Gew.%, bezogen auf die Masse der anwendungsfertigen Zubereitung vorliegt.

11. Verwendung nach Anspruch 9, wobei der erste Wirkstoff gemäß der Formel III und der zweite Wirkstoff gemäß der Formel IV in einem Massenverhältnis von 55:45 bis 80:20 in der anwendungsfertigen Zubereitung vorliegt.

12. Verwendung nach einem der vorangehenden Ansprüche, wobei die Zubereitung neben dem mindestens einen Wirkstoff bzw. der Wirkstoffmischung noch Zusätze zur topischen Anwendung enthält.

13. Verwendung nach Anspruch 12, wobei als Zusatz mindestens ein Phospholipid, insbesondere mindestens ein Soja-Phospholipid, verwendet wird.

14. Verwendung nach Anspruch 13, wobei das mindestens eine Phospholipid in einer Konzentration zwischen 1 Gew.% und 25 Gew.%, bezogen auf die Masse der anwendungsfertigen Zubereitung, verwendet wird.

15. Verwendung nach einem der Ansprüche 12 bis 14, wobei als weitere Zusätze Verdickungsmittel, Lösungsmittel, Öle, Fette, Wachse, Emulgatoren und/oder Puffer eingesetzt werden.
